# EUROPEAN PATENT APPLICATION

(11) **EP 3 536 326 A1**
(43) Date of publication of application: **11.09.2019**
(21) Application number: 19161569.9
(22) Date of filing: 08.03.2019
(51) Int. Cl.: A61K 31/718, A61K 31/733, A61P 3/04

(54) **DIETARY FIBER COMPOSITIONS FOR CURATIVE OR PROPHYLACTIC TREATMENT OF OBESITY AND OTHER CONDITIONS**

(30) Priority: 08.03.2018 EP 18160716
(71) Applicant: Coöperatie Koninklijke Cosun U.A., 4814 NE Breda (NL); Universiteit Maastricht, 6211 LK Maastricht (NL)
(72) Inventor: BLAAK, Ellen E., 6211 LK Maastricht (NL); CANFORA, Emanuel Enzo, 6211 LK Maastricht (NL); VAUGHAN, Elaine E., 4814 NE Breda (NL)
(74) Representative: Nederlandsch Octrooibureau

(57) **Abstract**

The present invention relates to dietary fiber compositions that can provide an increased level of acetate in the distal colon. Increase levels of acetate (or other short chain fatty acids; SCFA's) in the distal part of the colon is believed to be beneficial in the treatment of various diseases or conditions, such as being overweight or obese. To accomplish this using dietary intervention, which is clearly the preferred (first-line) option for the treatment and/or prevention of obesity (and related disorders), has been a challenge. It was now surprisingly found that combinations of an inulin-type fructan and resistant starch can be orally consumed to substantially increase the level of acetate in the distal part of the colon in human subjects. Hence, the present invention provides dietary fiber products comprising combinations of an inulin-type fructan and resistant starch, alimentary products comprising such dietary fiber compositions, as well as the use of the dietary fiber compositions or the products containing them in the treatment or prevention of a medical or non-medical condition, such as obesity or being overweight.

## Description

### Field of the invention

The present invention relates to dietary fiber compositions that can provide an increased level of acetate in the distal colon. According to another aspect the present invention relates to the use of such dietary fiber compositions for curative and/or prophylactic treatment of subjects in need thereof, e.g. subjects suffering from or at risk of suffering from a disease or disorder wherein increasing the level of acetate in the distal colon may be beneficial. According to yet another aspect the present invention relates to alimentary products comprising the dietary fiber compositions.

### Background of the invention

Obesity is a major health problem in developed countries, and is rapidly becoming a significant health problem in developing countries, where it is reaching epidemic proportions. In the adult population in the US, one out of three adults is obese.

Obesity is a complex, multifactorial disease. Obesity is typically defined quite simply as excess body weight for height, but this simple definition belies an etiologically complex phenotype primarily associated with excess adiposity, or body fatness, that can manifest metabolically and not just in terms of body size. Excessive adipose tissue is associated with a number of chronic diseases including hyperlipidemia, high blood pressure, glucose/carbohydrate intolerance and diabetes and cardiovascular diseases. The precise association between obesity and these chronic diseases is poorly understood, but solid epidemiological data support the role of fat mass. The number of adipocytes in a given fat mass increases in obesity and depends on both hypertrophy of preexisting adipocytes and hyperplasia, the formation of new adipocytes (adipogenesis).

Adipose tissue, skeletal muscle and the liver are major organs involved in substrate metabolism. Research has revealed the presence of a strong inter-organ relationship in substrate metabolism. Functional disturbances in either of these organs can theoretically cause or contribute to the development of insulin resistance. The obese insulin resistant state is characterized by adipose tissue dysfunction, systemic lipid overflow and ectopic lipid accumulation in metabolically active organs such as the liver, pancreas and skeletal muscle. Additionally, skeletal muscle exhibits an impaired mitochondrial function and an impaired ability to adjust lipid oxidation to lipid supply. This leads to the accumulation, altered composition/localisation of bioactive lipid metabolites interfering with insulin action.

The gut microbiota is increasingly being recognized as an important factor in fat distribution, insulin sensitivity and glucose and lipid metabolism. Accordingly, the intestinal microbiota could play an important role in the development of obesity and diabetes. One of the important functions of the human microbiota is the fermentation of indigestible carbohydrates, i.e. dietary fiber or resistant starches. The major products of this fermentation process are the short-chain fatty acids (SCFA), acetate, propionate and butyrate.

Several *in vivo* rodent studies have shown that SCFA supplementation prevented diet-induced obesity and insulin resistance. Especially, increasing the acetate availability of the colonic and systemically most abundant SFCAs prevents diet-induced body weight gain, counteracts adiposity, and improves glucose homeostasis and insulin sensitivity.

For, example sodium acetate (5% (w/w)) added to high-fat fed mice for 12 weeks reduced body weight gain and improved insulin sensitivity without changing food intake or physical activity (Den Besten G, Bleeker A, Gerding A, van Eunen K, Havinga R, van Dijk TH, et al. Diabetes. 2015, 64:2398-408).

Another study showed that dietary supplementation of acetate (5% (w/w)) for 12 weeks to high-fat fed mice inhibited body weight gain, reduced plasma glucose and insulin concentrations, as well as reduced pro-inflammatory cytokines and chemokines (Lu Y, Fan C, Li P, Lu Y, Chang X, Qi K. Scientific Reports. 2016, 6:37589). However, data from rodents are conflicting and indicate that an increased acetate turnover promotes body weight gain and insulin resistance (Perry RJ, Peng L, Barry NA, Cline GW, Zhang D, Cardone RL, Petersen KF, Kibbey RG, Goodman AL, Shulman GI. Nature. 2016 534,7606:213). A reason for these controversies may be related to the mode and site of acetate administration, as well as to the species and the metabolic phenotype of animals used (Canfora EE, van der Beek CM, Jocken JWE, Goossens GH, Holst JJ, Olde Damink SWM, Lenaerts K, Dejong CHC, Blaak EE. Sci Rep. 2017, 7:2360).

An acute study wherein acetate administration to both the proximal as well as the distal colon of healthy, overweight men showed that fat oxidation was significantly increased, when acetate was administered in the distal part of the colon. In contrast, no effect on energy expenditure or substrate oxidation was seen when acetate was administered in the proximal colon (Canfora EE, van der Beek CM, Jocken JWE, Goossens GH, Holst JJ, Olde Damink SWM, Lenaerts K, Dejong CHC, Blaak EE. Sci Rep. 2017, 7:2360).

Thus, the SCFAs consumed that may enter the small intestine or proximal colon, or that are generated by gut microbiota fermentation in the proximal colon, cannot give this beneficial effect on fat oxidation and energy expenditure in humans; the SCFAs must be administered or generated in sufficient concentrations in the distal human colon to give the beneficial effect.

Since dietary intervention is clearly the preferred (first-line) option for the treatment and/or prevention of obesity (and related disorders) the challenge is to provide a composition that, on the one hand, has the capability of delivering/liberating sufficient quantities of SCFA in the distal part of colon and, on the other hand, can be incorporated into the diet in adequate quantities without negative effects, for example on taste (of food) or necessity to use capsules.

It is an objective of the present invention to provide such compositions as well as the associated treatments.

### Summary of the Invention

It was now surprisingly found that specific combinations of dietary fibers, notably a combination of an inulin-type fructan and resistant starch can be orally consumed to substantially increase the level of acetate in the distal part of the colon in human subjects.

As will be illustrated in the experimental part below, the present inventors, in a validated *in vitro* human intestinal model (TIM) study, have established that such combinations of resistant starch and inulin show significantly increased acetate levels in the distal colon. A human study was designed and conducted supporting these findings.

Inulin-type fructans has been shown to be fermented to mainly acetate, and to some propionate and butyrate *in vivo* or based on analysis of fecal samples (Boets E, Deroover L, Houben E, Vermeulen K, Gomand SV, Delcour JA, Verbeke K. Nutrients. 2015 7:8916-29.). However, the specific site of inulin fermentation within the colon, correlation to SCFAs produced at that site and relationship with obesity have not been elucidated.

In the literature, the effects of certain inulin and inulin-derived products, mostly short-chain inulin, and inulin, and combinations of short-chain and long-chain inulin, on changes in body weight have been studied.

Most studies reported no significant effects on body weight: Forcheron F, Beylot M. Metabolism. 2007 56:1093 with 10 g/d of an oligofructose and inulin mixture for 180 days; Tripkovic L, Muirhead NC, Hart KH, Frost GS, Lodge JK, J Hum Nutr Diet. 2015 28:476 with 15 g inulin per day for 4 weeks in males with higher risk of cardiovascular disease; Tovar AR, Caamaño Mdel C, Garcia-Padilla S, Garcia OP, Duarte MA, Rosado Nutr J 2012 11:44 with 10 g/d inulin for 90 days in obese women; Liber A, Szajewska H. Br J Nutr. 2014 112:2068-74 in in overweight and obese children; Reimer RA, Willis HJ, Tunnicliffe JM, Park H, Madsen KL, Soto-Vaca A. Reimer Mol Nutr Food Res. 2017 61(11) with 16 g/d oligofructose for 84 days; Verhoef SP, Meyer D, Westerterp KR Br J Nutr. 2011 106:1757-62 using 10 or 16 g daily in normal-weight and overweight men and women; amongst others showed no statistically significant effect. Importantly, studies with very high doses such as 30 gram of oligofructose per day in overweight and obese volunteers could not affect body weight or adiposity (Daud NM, Ismail NA, Thomas EL, Fitzpatrick JA, Bell JD, Swann JR, Costabile A, Childs CE, Pedersen C, Goldstone AP, Frost GS. Obesity (Silver Spring). 2014 22:1430-8).

However other studies showed significant effect on body weight: Genta S Cabrera W, Habib N, Pons J, Carillo IM, Grau A, Sanchez S Clin Nutr. 2009 28(2):182 with 12.5 g/d yacon syrup, a source of fructooligosaccharides, for 120 days obese and slightly dyslipidemic women; Dehghan P, (Pourghassem Gargari B, Asghari Jafar-abadi M. Nutrition. 2014 30:418-23, using 10g/d of inulin for 8 weeks in female diabetic patients; Parnell JA, Reimer RA. Br J Nutr. 2010 103:1577-84, in healthy adults who consumed 21g/d of oligofructose for 12 weeks, and Guess ND, Dornhorst A, Oliver N, Bell JD, Thomas EL, Frost GS. Nutr Metab (Lond). 2015 24;12:36 in pre-diabetic subjects receiving dietary guidance to lose weight who consumed 30g/d of inulin. Two studies in children also showed a beneficial effect of 8g/d oligofructose-enriched inulin on body weight (Abrams SA, Griffin IJ, Hawthorne KM, Ellis KJ. J Pediatr. 2007 151:293-8) (Nicolucci AC, Hume MP, Martinez I, Mayengbam S, Walter J, Reimer RA. Gastroenterology. 2017 153:711).

Thus, outcomes of different studies with inulin show conflicting effects on body weight. In addition, high doses of inulin (>20 g/day) can cause gastrointestinal side effects, which may give compliance issues.

Without wishing to be bound by any theory, the present inventors currently believe that the positive results obtained in their present work can be attributed to the fact that the colonic microbiota first ferment the resistant starch as an energy source. Thus the inulin, especially the inulin with longer chains, can reach the distal colon where it is fermented by other gut microbes which produce the SCFA acetate at that region, whereby the acetate may exert beneficial effects locally and/or may enter the systemic circulation, consequently leading to its metabolic effects.

Hence, the present invention provides for the first time provides dietary fiber products comprising combinations of an inulin-type fructan and resistant starch, alimentary products comprising such dietary fiber compositions, as well as the use of the dietary fiber compositions or the products containing them in the treatment or prevention of a medical or non-medical condition, such as obesity or being overweight.

### Detailed Description of the Invention

Hence, a first aspect of the present invention is directed to a dietary fiber composition comprising a combination of (a) one or more inulin-type fructans and (b) resistant starch.

The word "combination" as used in the definition of the compositions typically means that an inulin-type fructan and a resistant starch are comprised within one single composition. The invention provides the insight that both components are needed to achieve an optimal or maximum or significant or measurable release of SCFA in the distal part of the colon. As will be understood by those skilled in the art, the benefits of the present invention may also be accomplished by sequential or simultaneous administration of two separate compositions each comprising one of the two components. Such uses and methods of treatment are also within the scope of the present invention. However it is contemplated that the co-administration of the combination as part of a single composition of product is particularly advantageous.

As used herein the term "inulin-type fructan" is a generic term to cover all β(2,1) linear fructans. Inulin is a term generally used to denote naturally occurring polysaccharides consisting of chain-terminating glucosyl moieties and a repetitive fructosyl moiety, which are linked by β(2,1) bonds. The correct chemical nomenclature for inulin is poly-β-D-(2→1)-fructofuranosyl-α-D-glucopyranose. Native inulin is a heterogeneous collection of such oligomers and polymers. Native inulin may be processed in various ways to change the physical and/or chemical characteristics thereof. Chemical or enzymatic hydrolysis may be used for example to remove the end glucose and/or to shorten the (average) chain length of the fructan polymers and/or oligomers.

Inulin and inulin derived polymers and oligomers can be characterized by the degree of polymerization (DP), which is a parameter expressing the number of fructose units in the fructose polymer or oligomer.

Native chicory inulin mainly comprises molecules having a degree of polymerisation (DP) 2 to 60 and the average degree of polymerization (DPav) of native inulin typically may be around 12. Its partial enzymatic hydrolysis product is oligofructose (DP 2 to 8; DPav = 4), and by applying specific separation technologies a long-chain inulin (DP 10 to 60; DPav = 25) can be produced. Finally, a specific product known as oligofructose-enriched inulin is obtained by combining chicory long-chain inulin and oligofructose.

An estimation of the length of inulin chains present in for example chicory inulin can be made from an inulin chain length distribution analysed for example by a chromatographic method (HPAEC-PAD).

In accordance with an embodiment of the invention, it is preferred that the at least one inulin-type fructan is a long-chain inulin. Long-chain inulin may also typically be referred to as 'inulin', 'fructan polymer', etc. Long-chain inulin is generally obtained by crystallization and removal of impurities. Long-chain inulin therefore can comprise fructose polymer made of poly-β-D-(2→1)-fructofuranosyl-α-D-glucopyranose but also derivatives thereof such as poly-β-D-(2→1)polyfructofuranose. In accordance with the invention, the long-chain inulin is typically characterized by the fact that most of the molecules have a DP within a certain range. Preferably the lower limit of said range is 8, 9, 10, 11, 12, 13, 14, 15, 16, 17, 18, 19, 20, 21, 22, 23, 24 or 25. Preferably the upper limit of said range is 60, 55, 50, 45, 40, 37, 35, 34, 33, 32, 32, 30 or 29. In an embodiment of the invention at least 60%, at least 70%, at least 80%, at least 90%, at least 95% or at least 99% of the molecules has a DP within the ranges as defined above, preferably within the range of 6-80. In a preferred embodiment of the invention the long-chain inulin is characterized by a degree of polymerization (DPav) of at least 10, at least 11, at least 12, at least 13, at least 14, at least 15, at least 16, at least 17, at least 18, at least 19 or at least 20. There is no particular upper limit. Typically, the long-chain inulin may have an average degree of polymerization of less than 60, less than 55 or less than 50. In a preferred embodiment of the invention the long-chain inulin is characterized by an average degree of polymerization average DP within the range of 20-30, preferably 22-29, e.g. 20, 21, 22, 23, 24, 25, 26, 27, 28, 29 or 30.

A preferred long-chain inulin is Frutafit®TEX! from Sensus, Roosendaal, the Netherlands. Frutafit®TEX! is a natural powdered food ingredient based on or derived from native chicory inulin, and known for its texturizing properties. In order to obtain Frutafit®TEX!, chicory roots are first pretreated and subsequently the obtained mixture is purified. The pretreatment may comprise a washing, a slicing and extraction steps. The extraction step is preferably carried out using only hot water. Hot water extraction of inulin from chicory takes place at similar conditions of extraction of sugar from sugar beets. It means that preferably no other solvent is being used. Subsequently long-chain inulin is generally obtained by crystallization and removal of impurities (Meyer & Blaauwhood, 2009, edited by G.O. Philips and P.A. Williams, pages 829-848, Woodhead, Cambridge, UK). The DP of the molecules in the Frutafit®TEX! product is mainly within the range of 8 to 60.

In accordance with an embodiment of the invention, the at least one inulin-type fructan is a short-chain inulin. Short-chain inulin may be produced from inulin by partial enzymatic hydrolysis or by physical methods such as filtration. In accordance with the invention, the short-chain inulin is typically characterized by the fact that most of the molecules have a DP within a certain range. Preferably the lower limit of said range is 2, 3, 4, 5, 6, 7, 8, 9 or 10. Preferably the upper limit of said range is 35, 30, 25, 22, 20, 19, 18, 17, 16, 15, 14, 13, 12, 11 or 10. In an embodiment of the invention at least 60%, at least 70%, at least 80%, at least 90%, at least 95% or at least 99% of the molecules has a DP within the ranges as defined above, preferably within the range of 2-35. In a preferred embodiment of the invention the short-chain inulin is characterized by an average degree of polymerization (DPav) of at least 2, at least 3, at least 4, at least 5, at least 6, at least 7, at least 8, at least 9, or at least 10. In a preferred embodiment of the invention the short-chain inulin is characterized by a DPav of less than 20, less than 17, less than 15, less than 14, less than 13, less than 12, less than 11, less than 10, less than 9 or less than 8. In a preferred embodiment of the invention the short-chain inulin is characterized by an average degree of polymerization DPav within the range of 6-10 or 7-9, e.g. a DPav of 6, 7, 8, 9 or 10.

In another preferred embodiment of the invention the at least one inulin-type fructan is oligofructose or fructo-oligosaccharide (FOS), which may also be considered a short-chain inulin. In accordance with the invention, the oligofructose or FOS is typically characterized by the fact that most of the molecules have a DP within a certain range. Preferably the lower limit of said range is 2, 3, 4 or 5. Preferably the upper limit of said range is 15, 14, 13, 12, 11, 10, 9 or 8. In an embodiment of the invention at least 60%, at least 70%, at least 80%, at least 90%, at least 95% or at least 99% of the molecules in has a DP within the ranges as defined above, preferably within the range of 2-8. In a preferred embodiment of the invention the oligofructose or FOS is characterized by an average degree of polymerization (DPav) of at least 2, at least 3 or at least 4. In a preferred embodiment of the invention the inulin-type fructan is characterized by a DPav of less than 10, less than 9 or less than 8. In a preferred embodiment of the invention the oligofructose or FOS is characterized by an average degree of polymerization DPav within the range of 2-8, e.g. a DPav of 2, 3, 4, 5, 6, 7 or 8.

Embodiments are envisaged wherein the inulin-type fructan comprises a combination of different inulin-type fructan products, such as a combination of a short chain inulin product and a long chain inulin product.

In accordance with an embodiment the invention, it is preferred that the at least one inulin-type fructan is native inulin. A preferred native inulin is chicory (*Cichorium intybus*) inulin. Other sources for inulin production include artichoke and agave inulin, such as native chicory inulin, is a polydisperse mixture of linear oligomers and polymers with a DP within the range of 2-60. For native inulin, the DP's and DPav can vary widely. A suitable example of a native inulin product that can be used in accordance with the invention is Frutafit® HD, which is a natural powdered food ingredient extracted from chicory roots and is available from Sensus, Roosendaal, the Netherlands.

The second component of the dietary fiber compositions of the invention is a resistant starch. Starches are polysaccharides composed of a number of monosaccharides or sugar (glucose) molecules linked together with α-D-(1-4) and/or α -D-(1-6) linkages. Starch consists of 2 main structural components, the amylose, which is essentially a linear polymer in which glucose residues are α -D-(1-4) linked typically constituting 15% to 20% in most starches, and amylopectin, which is a larger branched molecule with α -D-(1-4) and α -D-(1-6) linkages and is the major component of most starches.

Starches can be classified according to their behavior when incubated with enzymes without prior exposure to dispersing agents. According to this system starches can be classified as rapidly digestible starch (RDS), slowly digestible starch (SDS) and resistant starch (RS).

The term "resistant starch" was first coined by Englyst and others (1982) to describe a small fraction of starch that was resistant to hydrolysis by exhaustive α-amylase and pullulanase treatment *in vitro.* RS is the starch not hydrolyzed after 120 min of incubation. However, because starch reaching the large intestine may be more or less fermented by the gut microflora, RS is now defined as that fraction of dietary starch, which escapes digestion in the small intestine. It is measured chemically as the difference between total starch (TS) obtained from homogenized and chemically treated sample and the sum of RDS and SDS, generated from non-homogenized food samples by enzyme digestion (RS = TS - (RDS + SDS)). Resistant starch is usually further classified as RS1, RS2, RS3 or RS4.

RS1 refers to starch that is physically inaccessible as it is locked within cell walls of botanical substances and therefore qualifies as resistant starch. The RS1 type of resistant starch is, for example, found in partially milled grains, seeds, and legumes. RS1 is heat stable in most normal cooking operations and enables its use as an ingredient in a wide variety of conventional foods.

RS2 refers to native resistant starch which is a component of starch granules such as those found in bananas (especially green bananas) and raw potatoes. Bananas and raw potatoes have relatively low gelatinization temperatures, typically on the order of about 60 °C to about 80 °C, which often presents substantial problems in the formulation of food products. Intragranular polymeric rearrangements that lead to an increased granular resistance to amylase digestion are also included in this category. This increased resistance could be the result of heat and/or moisture treatments or annealing of the intact granule. It is measured chemically as the difference between the glucose released by the enzyme digestion of a boiled homogenized food sample and that from an unboiled, non-homogenized food sample. In raw starch granules, starch is tightly packed in a radial pattern and is relatively dehydrated. This compact structure limits the accessibility of digestive enzymes, various amylases, and accounts for the resistant nature of RS2 such as, ungelatinized starch. In the diet, raw starch is consumed in foods like banana. RS1 and RS2 represent residues of starch forms, which are digested very slowly and incompletely in the small intestine.

RS3 refers to retrograded non-granular starch or crystalline non-granular starch, such as starch found in cooked and cooled potatoes, bread crusts, and cereals (cornflakes, for example) and starch pastes that have been extensively processed (by repeated cooking and cooling). It is measured chemically as the fraction, which resists both dispersion by boiling and enzyme digestion. It can only be dispersed with KOH or dimethyl sulphoxide. RS3 is entirely resistant to digestion by pancreatic amylases.

RS4 refers to specific starches that have been chemically modified and/or re-polymerized (which may include molecular weight reduction), such as ethers, esters, and cross-bonded starches, as well as chain linkage altered dextrins, pyrodextrins, and maltodextrins.

The resistant starch that is utilized in the present invention may be any resistant starch, such as any of the RS1, RS2, RS3, or RS4 resistant starches or any combination of resistant starches. Preferably, the resistant starch that is utilized in the present invention is selected from RS2, RS3 and RS4, more preferably from RS2 and RS3. Most preferably the resistant starch is RS2.

In accordance with the invention, resistant starch can suitably be used that is derived from sources such as corn, wheat, rice, legume, pea, banana, barley, triticale, sorghum, milo, cassava, oat, potato, tapioca, sago, ocarina, etc. In a preferred embodiment of the invention, the resistant starch is derived from corn, potato or banana, more preferably from corn or potato.

In an embodiment of the invention, the resistant starch is selected from the group consisting of resistant waxy maize starch; resistant regular or normal maize starch; resistant wheat starch; resistant rice starch; resistant legume, pea or pulse starch; resistant barley starch; resistant triticale starch; resistant sorghum starch; resistant milo starch; resistant cassava starch; resistant banana starch, resistant oat starch; resistant potato starch; resistant tapioca starch; and resistant sago starch, more preferably from the group consisting of resistant corn starch, resistant potato starch and resistant banana starch, most preferably from resistant corn starch and resistant potato starch.

In embodiments of the invention, the resistant starch is characterized by its specific amylose content. In one embodiment of the invention the resistant starch is characterized by a high amylose content, such as an amylose content, based on the total dry weight of the starch, of at least 35 wt.%, at least 37.5 wt.%, at least 40 wt.%, at least 42.5 wt.%, at least 45 wt.%, at least 47.5 wt.%, at least 50 wt.%, at least 52.5 wt.%, or at least 55 wt.%. There is no particular upper limit, although practically the resistant starch will have an amylose content, based on the total dry weight of the starch, of less than 75 wt.%, less than 70 wt.%, less than 65 wt.% or less than 60 wt.%. Currently, a high-amylose corn starch with the amylose content of up 70 wt.% is available.

Based on X-ray diffraction patterns, starches can be classified as type A, type B and type C. The type A structure has amylopectin of chain lengths of 23 to 29 glucose units. The hydrogen bonding between the hydroxyl groups of the chains of amylopectin molecules results in the formation of outer double helical structure. In between these micelles, linear chains of amylose moieties are packed by forming hydrogen bonds with outer linear chains of amylopectin. This pattern is very common in cereals. The type B structure consists of amylopectin of chain lengths of 30 to 44 glucose molecules with water inter-spread. This is the usual pattern of starches in raw potato and banana. the type C structure is made up of amylopectin of chain lengths of 26 to 29 glucose molecules, a combination of type A and type B, which is typical of peas and beans. One of the causes of resistance to enzymes is the crystallinity of native type B starch granules. In accordance with the present invention, it is preferred that the resistant starch is a type B (resistant) starch.

In embodiments of the invention, the resistant starch is characterized by its specific granule size distribution. In an embodiment of the invention, the resistant starch is characterized by a volume-weighted mean diameter D[4,3] of at least 5 µm, at least 7.5 µm, at least 10 µm, at least 12.5 µm, at least 15 µm, at least 17.5 µm, at least 20 µm, at least 22.5 µm or at least 25 µm. In an embodiment of the invention, the resistant starch is characterized by a volume-weighted mean diameter D[4,3] of less than 150 µm, less than 100 µm, less than 75 µm, less than 50 µm, less than 40 µm, less than 35 µm, less than 30 µm, or less than 25 µm.

In embodiments of the invention resistant starch is a high amylose maize starch. Some suitable, though non-exhaustive, examples of high amylose starch are the HI-MAIZE™ high amylose starches (ex Ingredion, Westchester, USA), such as HI-MAIZE® 260.

In embodiments of the invention, the resistant starch is granular potato starch. A suitable example thereof includes type 2 resistant starch Potato Starch Food Grade Quality, which is derived from potatoes; it is a granulated, light beige powder, is intended for use in food and has a GRAS status (ex AVEBE, Veendam, The Netherlands).

In embodiments of the invention, the resistant starch is resistant tapioca starch. A suitable example thereof includes C* Actistar 11700 (ex Cerestar, France).

In a preferred embodiment of the invention, a dietary fiber composition as defined herein before is provided, wherein the inulin-type fructan (a) and the resistant starch (b) are present in a ratio (w/w) of at least, 1/5, at least 1/4, at least 1/3 at least 1/2 or at least 1/1. In a preferred embodiment of the invention, a dietary fiber composition as defined herein before is provided, wherein the inulin-type fructan (a) and the resistant starch (b) are present in a ratio (w/w) of 10 or less, 7 or less, 5 or less, 4 or less, 3 or less, or 2 or less. In a preferred embodiment of the invention, a dietary fiber composition as defined herein before is provided, wherein the inulin-type fructan (a) and the resistant starch (b) are present in a ratio (w/w) within the range of 1/5 to 10/1, preferably 1/2 to 5/1, more preferably 1/1to 2/1.

A second aspect of the invention is directed to, stated generally, the prophylactic or curative treatment of a medical condition or pathology in a subject in need thereof, using the fiber composition of the present invention. Based on the information provided for the first time herein, it is contemplated that the dietary fiber compositions of the present invention are useful for treatment of a disease or medical condition, especially a disease or condition that may benefit from increases in the SCFA content in the distal part of the colon, such as obesity. The method comprises providing to the subject a composition in accordance with any of those described herein in an amount effective to treat or prevent the disease or medical condition.

More in particular, in one embodiment, the invention provides a dietary fiber composition comprising a) an inulin-type fructan comprising fructose polymers (inulin) and/or fructooligosaccharides; and (b) resistant starch, or a product containing said dietary fiber composition, as defined herein for use in the prophylactic or curative treatment of a disease or condition, especially a disease or condition that may benefit from increases in the SCFA content in the distal part of the colon, such as obesity, in a subject in need thereof.

In one embodiment, the invention provides an inulin-type fructan as defined herein for use in the prophylactic or curative treatment of a disease or condition, especially a disease or condition that may benefit from increases in the SCFA content in the distal part of the colon, such as obesity, in a subject in need thereof, wherein the treatment further comprises the administration to said subject of a resistant starch.

In one embodiment, the invention provides a resistant starch as defined herein for use in the treatment and/or prevention of a disease or condition, especially a disease or condition that may benefit from increases in the SCFA content in the distal part of the colon, such as obesity, in a subject in need thereof, wherein the treatment further comprises the administration to said subject of an inulin-type fructan.

More in particular, in one embodiment, the invention provides a method of treating and/or preventing a disease or condition, especially a disease or condition that may benefit from increases in the SCFA content in the distal part of the colon, such as obesity, in a subject in need thereof, said method comprising administering to said subject a dietary fiber composition comprising a) an inulin-type fructan; and (b) resistant starch, or a product containing said dietary fiber composition, as defined herein.

In accordance with the embodiments as defined here above, the term "curative treatment", in relation a given disease or condition, includes, but is not limited to, arresting the development of the disease or condition; relieving the disease or condition, causing regression of the disease or condition; or relieving a symptom caused by or resulting from the disease or condition.

In accordance with the embodiments as defined here above, the term "prophylactic treatment" in relation to a given disease or condition means preventing the onset of disease development if none had occurred, or preventing the disease or condition from occurring in a subject that may be predisposed to the disease or condition but has not yet been diagnosed as having the disease or condition.

As already indicated herein before, the present invention resides in the finding that the (oral) administration of the dietary fiber composition of the invention to a human subjects results in an increase in the SCFA levels in the distal part of the colon. Such an increase in SCFA levels is understood to be beneficial to a variety of diseases or conditions, including, in particular, obesity and related diseases and conditions, as well as a number of other diseases or conditions, such as certain digestive diseases or conditions or diseases or conditions of the intestinal tract.

Hence, in one embodiment of the invention, the disease or condition is obesity and/or a disease or condition associated with obesity. The term "obesity", as used herein, means an excess of adipose tissue in the body. In this connection, obesity is fundamentally to be seen as the increased level of fatness, which leads to a health risk. The health risk accompanying obesity is presumed to rise continuously as the level of fatness increases. Obesity is generally defined by body mass index (BMI), which is defined as the body weight measured in kilograms divided by the height (in meters) squared. According to the most commonly used definitions, established by the World Health Organization (WHO) in 1997 and published in 2000, a BMI in excess of 30 is indicative of obesity.

In some embodiments, the obesity is prevalent obesity. In some embodiments, the obesity is prevalent abdominal obesity. In some embodiments, the obesity is new-onset obesity.

Because obesity is associated with the onset or progression of other diseases, the methods of the invention are further useful in methods of reducing complications associated with obesity including vascular disease, such as coronary artery disease, stroke, peripheral vascular disease, ischemia reperfusion, etc.; hypertension; insulin resistance, pre-diabetes, onset and/or progression of diabetes type II; hyperlipidemia and musculoskeletal diseases. The present invention, in various embodiments, accordingly provides methods of treating or preventing these obesity-associated diseases or conditions.

In some embodiments, the disease or condition is hyperglycemic condition. In certain embodiments, the hyperglycemic medical condition is insulin resistance, pre-diabetes, diabetes, diabetes mellitus type I, diabetes mellitus type II, or gestational diabetes, either insulin-dependent or non-insulin-dependent.

Metabolic syndrome is a cluster of conditions that synergistically increase the risk of cardiovascular disease, type 2 diabetes, and premature mortality. The components are abdominal obesity, impaired glucose metabolism, dyslipidemia, and hypertension. Prediabetes, which is a combination of excess body fat and insulin resistance, is considered an underlying etiology of metabolic syndrome. Prediabetes manifests as impaired fasting glucose and/or impaired glucose tolerance. The invention provides a method of preventing or treating Metabolic Syndrome, or reducing one, two, three or more risk factors thereof, in a subject. In some embodiments of the invention the disease or condition is overweight or an unhealthy weight. Hence in an embodiment of the invention the method is a method of controlling weight, a method of losing weight, a method of reducing weight, a method of preventing weight gain, a method of limiting weight gain, a method of reducing overweight, a method of preventing overweight, a method of inducing weight loss, a method of increasing weight loss, a method of managing weight and/or a method of maintaining a healthy weight. In an embodiment of the invention, the method is a method of losing weight within a predetermined interval, e.g. to lose weight within 12 months, within 6 months, within 4 months, within 3 months, within 2 months, within 1 month, within 4 weeks, within 3 weeks, within 2 weeks or within 1 week.

In some embodiments of the invention the disease or condition is excess body fat (mass), which may also be referred to as adiposity. Hence in an embodiment of the invention the method is a method of controlling body fat (mass), a method of losing body fat (mass) a method of reducing body fat (mass), a method of preventing a gain in body fat (mass), a method of limiting gain in body fat (mass), a method of reducing body fat (mass), a method of preventing excess body fat (mass), a method of inducing loss of body fat (mass), a method of increasing loss of body fat (mass), a method of managing body fat (mass) and/or a method of maintaining a healthy body fat (mass).In an embodiment of the invention, the method is a method of losing body fat (mass) within a predetermined interval, e.g. to lose body fat (mass) within 12 months, within 6 months, within 4 months, within 3 months, within 2 months, within 1 month, within 4 weeks, within 3 weeks, within 2 weeks or within 1 week.

Other embodiments are envisaged wherein the disease or disorder is a digestive condition, such as a functional gastrointestinal disorders (FGID). FGIDs are diagnosed and classified using the Rome IV criteria (released in May 2016) and include functional bowel disorders such as functional diarrhea, functional constipation, Irritable Bowel Syndrome (IBS), e.g. IBS with predominant diarrhea [IBS-D], IBS with predominant constipation [IBS-C], and IBS with mixed bowel habits.

Other embodiments are envisaged wherein the disease or disorder is low-grade inflammation, e.g. chronic low-grade inflammation. Clinically low-grade inflammation is defined as a two to four-fold elevation in circulating levels of proinflammatory and anti-inflammatory cytokines as well as numerous other markers of immune system activity. Chronic low-grade inflammation plays a role in the pathology of numerous age-related chronic conditions.

The term "subject" as used herein refers to an animal, in particular a human, that is treatable by the method of the invention. The term "subject" refers to both the male and female gender unless one gender is specifically indicated. In accordance with the invention, the subject is a human subject. The human subject can be any age, e.g. the human subject can be a juvenile, an adolescent, an adult or an elderly subject. In embodiments of the invention the human subject is at least 18 years of age, preferably at least 25 years, at least 30 years, at least 35 years, at least 40 years, at least 45 years, at least 50 years, at least 55 years, at least 60 years or at least 65 years of age. There is no particular upper limit although in practice, human subjects treated in accordance with the invention will typically be less than 100 years of age, e.g. less than 95 or less than 90 years of age.

In accordance with embodiments of the invention, the subject in need thereof is a subject suffering from or at risk of suffering from any one of the diseases or conditions as defined here above.

Hence, in accordance with embodiments of the invention, the subject in need thereof is a subject suffering from or at risk of suffering from overweight, obesity, prevalent obesity, prevalent abdominal obesity or new-onset obesity.

In one embodiment, the subject is obese and/or suffering from complications associated with obesity.

In one embodiment, the subject is suffering from or at risk of suffering from complications associated with obesity, such as a subject suffering from one or more complications selected from vascular disease, such as coronary artery disease, stroke, peripheral vascular disease, ischemia reperfusion, etc.; hypertension; onset and/or progression of diabetes type II; hyperlipidemia and musculoskeletal diseases.

In one embodiment, the subject is suffering from or at risk of suffering from a hyperglycemic medical condition, such as diabetes, diabetes mellitus type I, diabetes mellitus type II, or gestational diabetes, either insulin-dependent or non-insulin-dependent.

In one embodiment, the subject is suffering from or at risk of suffering from metabolic syndrome. In one embodiment, the subject meets the criteria of either or both of the criteria set forth by the 2001 National Cholesterol Education Program Adult Treatment Panel or the WHO, that individual is considered as afflicted with Metabolic Syndrome, as defined here above.

In one embodiment, the subject has a body mass index (BMI) of 25 or more, e.g. 26 or more, 27 or more, 28 or more, 29 or more, 30 or more, 31 or more, 32 or more, 33 or more, 34 or more, 35 or more, 36 or more, 37 or more, 38 or more, 39 or more or 40 or more.

In one embodiment, the subject is a prediabetic subject. The World Health Organization (WHO) has defined prediabetes as a state of intermediate hyperglycemia using two specific parameters, impaired fasting glucose (IFG) defined as fasting plasma glucose (FPG) of 6.1-6.9 mmol/L (110 to 125 mg/dL) and impaired glucose tolerance (IGT) defined as 2 h plasma glucose of 7.8-11.0 mmol/L (140-200 mg/dL) after ingestion of 75 g of oral glucose load or a combination of the two based on a 2 h oral glucose tolerance test (OGTT). In one embodiment of the invention, the subject is suffering from impaired fasting glucose, meaning that the subject's blood sugar levels during fasting are consistently above the normal range, but below the diagnostic cut-off for a formal diagnosis of diabetes mellitus. In one embodiment, the subject has a blood sugar level during fasting within the range of 6.1-6.9 mmol/L. In one embodiment, the subject has a two-hour glucose level of 7.8 to 11.0 mmol/L on the 75-g oral glucose tolerance test.

In embodiments of the invention, the subject is a healthy subject, e.g. when the method is mainly carried out for prophylaxis. In embodiments of the invention, the subject is a non-overweight subject. In embodiments of the invention, the subject has a BMI of less than 25, less than 22, less than 20, less than 19, less than 18, less than 17, less than 16 or less than 15. In an embodiment, the subject has a blood sugar level during fasting below 6.1, below 6.0, below 5.9, below 5.8, below 5.7, below 5.6 or below 5.5 mmol/L. In some embodiment, the subject has a plasma glucose level below 7.8, below 7.6, below 7.5, below 7.4, below 7.3, below 7.2, below 7.1 or below 7.0 mmol/L on the 75-g oral glucose tolerance test.

One particularly preferred embodiment of the invention, provides a method of preventing, treating and/or slowing down the progression of pre-diabetes in a subject in need thereof, such as a subject that suffers from overweight or an obese subject, using the fiber composition of the present invention.

A further particularly preferred embodiment of the invention, provides a method of preventing, treating and/or slowing down the progression of diabetes type 2 in a subject in need thereof, such as a subject that suffers from overweight or an obese subject, using the fiber composition of the present invention.

A further particularly preferred embodiment of the invention, provides a method of treating and/or slowing down the progression of obesity in a subject in need thereof, such as a subject that suffers from overweight or an obese subject, using the fiber composition of the present invention.

A further particularly preferred embodiment of the invention, provides a method of preventing obesity in a subject in need thereof, such as a lean healthy subject or a subject that suffers from overweight, using the fiber composition of the present invention.

A further particularly preferred embodiment of the invention, provides a method of improving glucose tolerance in a subject in need thereof, such as a lean healthy subject, a subject that suffers from overweight or an obese subject, using the fiber composition of the present invention.

A further particularly preferred embodiment of the invention, provides a method of increasing energy expenditure in a subject in need thereof, such as a lean healthy subject, a subject that suffers from overweight or an obese subject, using the fiber composition of the present invention.

In embodiments of the invention, the methods as described herein comprise the step of identifying a subject that is in need of receiving treatment with the compositions of the invention and/or identifying a subject suffering from or at risk of suffering from any of the conditions as described here above and/or a subject meeting any of the above-described physiological characteristics as described here above.

The methods as described herein, comprise the administration to the subject in need thereof of a composition or product as described herein in an amount effective to prevent or treat one or more of the conditions or pathologies as defined herein.

Typically, the treatments entails the administration of the inulin-type fructan and the resistant starch in unit dose form, either in combination or separately. As explained herein elsewhere, such a unit dose may take any form, including the form of an alimentary product comprising the inulin-type fructan and/or the resistant starch, wherein the alimentary product is provided in the form of an individually packaged single serving, each serving comprising the inulin-type fructan and/or the resistant starch in unit dose amount.

In an embodiment, the unit dose amount of the inulin-type fructan is at least 1 g, at least 1.5 g, at least 2 g, at least 2.5 g, at least 3 g, at least 3.5 g, or at least 4 g. In an embodiment, the unit dose amount of the inulin-type fructan is less than 25 g, less than 20 g, less than 15 g, less than 12.5 g, less than 10 g, less than 9 g, less than 8 g, less than 7 g, less than 6 g or less than 5 g. In an embodiment, the unit dose amount of the inulin-type fructan is 3-8 g, preferably 4-8 g, more preferably 5-8 g. In an embodiment, the unit dose amount of the inulin-type fructan is 3-8 g, preferably 3-7 g, more preferably 3-6 g.

In an embodiment, the unit dose amount of the resistant starch is at least 1 g, at least 1.5 g, at least 2 g, at least 2.5 g, at least 3 g, at least 3.5 g, or at least 4 g. In an embodiment, the unit dose amount of the resistant starch is less than 25 g, less than 20 g, less than 15 g, less than 12.5 g, less than 10 g, less than 9 g, less than 8 g, less than 7 g, less than 6 g or less than 5 g. In an embodiment, the unit dose amount of the resistant starch is 2-8 g.

As will be understood by those skilled in the art, based on the present teachings, the inulin-type fructan and the resistant starch are administered orally in the form of a single composition or, alternatively are administered simultaneously or consecutively. In embodiments wherein the inulin-type fructan and the resistant starch are administered consecutively, it is preferred that the time in between the administration of the respective components is less than 5 hours, preferably less than 4 hours, less than 3 hours, less than 2 hours, less than 1 hour, less than 30 minutes, less than 20 minutes, less than 15 minutes, less than 10 minutes or less than 5 minutes.

Unit doses of the inulin-type fructan and the resistant starch are preferably administered at least once a week, preferably at least once every 3 days, at least once every other day, at least once daily. In preferred embodiments of the invention, the treatment comprises the daily administration of unit doses of the inulin-type fructan and the resistant starch, preferably once a day, twice a day, three times a day or four times a day, more preferably once or twice a day, most preferably once a day.

In accordance with the invention, the treatment as defined herein before, is preferably continued for a period of at least two weeks, preferably at least 3 weeks, at least 4 weeks, at least 1 month, at least 5 weeks, at least 6 weeks, at least 7 weeks, at least 8 weeks, at least 2 months, at least 3 months, at least 4 months, at least 5 months, or at least 6 months.

In accordance with a preferred embodiment of the invention, the treatment as defined herein before is continued for a period which is sufficient to attain a change in the subject's microbiome in the distal part of the colon as a consequence of the administration of the inulin-type fructan, especially in case the subject to be treated is of the obese phenotype. In particularly preferred embodiments the treatment as defined herein before is continued for a period which is sufficient to attain an enrichment in microbiotic species capable of fermenting the inulin-type fructan of the invention in the distal part of the colon. This may typically take at least 1 week, at least 2 weeks, at least 3 weeks or at least 4 weeks of treatment. In some embodiments of the invention, the treatment may comprise the administration, during said period needed to attain a change in the subject's microbiome, of one or more additional compositions that can aid in said change in the microbiome, such as probiotic compositions, especially compositions comprising live and/or viable bacteria that have the capability of fermenting the inulin-type fructan of the invention. In some embodiments such probiotic compositions may be formulated in such a way as to promote targeting of the bacteria to the distal part of the colon. As will be understood by those skilled in the art, based on the present teachings, it is particularly preferred that treatment in accordance with the invention is continued for some time after the moment that the change in the subject's microbiome is attained, e.g. for a period of at least two weeks, preferably at least 3 weeks, at least 4 weeks, at least 1 month, at least 5 weeks, at least 6 weeks, at least 7 weeks, at least 8 weeks, at least two 2 months, at least three 3 months, at least 4 months, at least 5 months, or at least 6 months after the change in the subject's microbiome is attained.

In preferred embodiments of the invention, the treatment comprises the administration of the inulin-type fructan in an average amount of 1-32 g per day, preferably in an average amount of 2-24 g per day, more preferably in an average amount of 4-16 g per day, e.g. approximately 12 g per day; and/or the administration of the resistant starch in an average amount of 2-24 g per day, preferably in an average amount of 3-16 g per day, more preferably 4-12 g per day, e.g. approximately 7.5 g per day, over a period of at least two weeks, preferably at least 3 weeks, at least 4 weeks, at least 1 month, at least two months, at least three months, at least 4 months, at least 5 months, or at least 6 months.

A third aspect of the invention is directed to stated, generally, the treatment and/or prevention of a non-medical condition in a subject in need thereof, using the fiber composition of the present invention. More in particular, in one embodiment, the invention provides a method of treatment and/or prevention of a non-medical condition, typically a method of treating or preventing being overweight, in a subject, said method comprising administering to said subject a dietary fiber composition comprising a) an inulin-type fructan; and (b) resistant starch, or a product containing said dietary fiber composition, as defined herein.

As will be understood by those skilled in the art, a subject can be overweight without such overweight causing any significant risks with regard to the subject's general health and/or well-being, while said subject may still desire to lose weight, e.g. for purely cosmetic reasons. Furthermore, a subject may be of normal or average weight, i.e. not overweight, and desire to prevent weight increase, e.g. for purely cosmetic reasons. Furthermore, healthy subjects may be treated in accordance with the invention in order to change non-medical digestive patterns, e.g. reduce flatulence, wind, intestinal bloating, distention of the abdomen by gas, abdominal cramps, rumbling, improve stool frequency or stool consistency or bowel function. Hence an aspect of the invention relates to a method of treating a healthy subject by the administration of the dietary fiber compositions of the invention and/or by the simultaneous or sequential administration of an inulin-type fructan and a resistant starch.

In one embodiment such method of treatment is carried out for non-medical reasons, e.g. for cosmetic purposes. Such treatment of healthy subjects for non-medical reasons will typically rely on the use of the same compositions and products as well as the same routes of administration and the same dosage regimens as defined here above in relation to medical treatments. In an embodiment of the invention, the subject is a healthy subject. In an embodiment of the invention, the subject is a non-overweight subject. In an embodiment of the invention, the subject has a BMI of less than 25, less than 22, less than 20, less than 19, less than 18, less than 17, less than 16 or less than 15. In an embodiment, the subject has a blood sugar level during fasting below 6.1, below 6.0, below 5.9, below 5.8, below 5.7, below 5.6 or below 5.5 mmol/L. In an embodiment, the subject has a plasma glucose level below 7.8, below 7.6, below 7.5, below 7.4, below 7.3, below 7.2, below 7.1 or below 7.0 mmol/L on the 75-g oral glucose tolerance test. Hence in an embodiment of the invention the method is a non-medical method of controlling weight, losing weight, reducing weight, preventing weight gain, limiting weight gain, inducing weight loss, increasing weight loss, managing weight and/or maintaining a healthy weight, in a healthy subject. In an embodiment of the invention, the method is a non-medical method of losing weight within a predetermined interval, e.g. to lose weight within 12 months, within 6 months, within 4 months, within 3 months, within 2 months, within 1 month, within 4 weeks, within 3 weeks, within 2 weeks or within 1 week.

A fourth aspect of the invention is directed to an alimentary product comprising the dietary fiber composition of the present invention. As used herein the term 'alimentary product' refers to products intended for oral consumption by a mammal, typically a human, so as to provide sustenance and/or satisfy nutritional requirements.

In a preferred embodiment of the invention, the alimentary product is a liquid, semi-solid or solid composition suitable for oral consumption by a mammal, typically a human, either in the form in which it is presented or after further processing, such as the addition of one more further edible components.

This alimentary product is preferably a food composition, a food product, a dietetic food product, a nutraceutical, a functional food product, a medical food product, a food additive, a food supplement, an infant food product, a follow-on formula, or a food product for elderly people.

As used herein, "nutraceuticals" generally encompass foods or food products that provide health and medical benefits. Nutraceuticals are edible and may be eaten directly by humans, but are preferably provided to humans in the form of additives or nutritional supplements, e.g., in the form of tablets or capsules of the kind sold in health food stores. Hence, in an embodiment of the invention, a nutraceutical product is provided, typically a product in the form of a tablet or a capsule, comprising the dietary fiber composition as defined herein. Alternatively, it is envisaged that the product is provided in the form of a sachet containing a powder comprising the dietary fiber composition. Preferably, such products are provided in unit dose form, i.e. in a form wherein each tablet, capsule or powder sachet contains a unit dose, as defined herein elsewhere.

As used herein, the term 'dietetic food product' refers to products intended to satisfy particular nutritional requirements of specific groups of the population. This category of products has gained a lot of interest over the past decades, with the Slim-fast and Modifast product lines representing very well-known examples. Such dietetic products typically are presented in the form of snacks and meal-replacement products, wherein the consumption of such products instead of regular snacks and meals will provide certain dietary benefits, such as reduced caloric intake and/or increased intake of beneficial dietary components. The most well-known and popular examples of such dietetic products include the snack bar type product and liquid meal-replacement products. Other examples include ready-meals, savoury snacks, bakery products and dessert-type products.

Hence, a preferred embodiment of the invention concerns a dietetic food product, preferably a product selected from the group consisting of bars, such as nutritional bars, energy bars, snack bars, cereal bars, bars for diabetics etc.; liquid products, such as nutritional drinks, diet drinks, liquid meal-replacers, sports drinks and other fortified beverages; dessert-type products, such as puddings, yoghurts; savoury snacks, such as chips/ tortillas, puffed and baked snacks, crackers, pretzels, savoury biscuits; and bakery products, such as muffins, cakes, biscuits and pasta, spaghetti.

The amount of the dietary fiber composition contained in a specific food product depends on the kind of food product, in particular its size and composition, as well as on the frequency and amount in which the product is or is supposed to be consumed.

In a particularly preferred embodiment of the invention, the dietetic food product is presented in the form of individually packaged single servings. The term "single serving" as used herein refers to a certain quantity and/or size of the product that is adequate for consumption as a single portion for a single person. Such products may be in a form that is ready-to-eat or ready-to-consume or it may be in a form that requires further processing, such as heating or addition of a quantity of hot or cold water.

In a particularly preferred embodiment of the invention, the dietetic food product is presented in the form of individually packaged single servings, wherein each serving contains at least 10 % of the amount of the average daily amount of the dietary fiber composition as defined herein elsewhere, more preferably at least 25 % or at least 50 % of said average daily amount, more preferably 10 %, 20 %, 25 %, 30 %, 40 %, 50 %, 60 %, 70 %, 80 %, 90 % or 100 % of said average daily amount.

In a particularly preferred embodiment of the invention, the dietetic food product is presented in the form of individually packaged single servings, wherein each serving contains a unit dose as defined herein elsewhere. Hence in an embodiment, the amount of the inulin-type fructan in one serving is at least 1 g, at least 2.5 g, at least 5 g, at least 6 g, at least 7 g, at least 8 g, at least 9 g or at least 10 g. In an embodiment, the amount of the inulin-type fructan in one serving is less than 25 g, less than 20 g, less than 15 g, less than 12.5 g, less than 10 g, less than 9 g, less than 8 g, less than 7 g, less than 6 g or less than 5 g. In an embodiment, the amount of the resistant starch in one serving is at least 1 g, at least 2.5 g, at least 5 g, at least 6 g, at least 7 g, at least 8 g, at least 9 g or at least 10 g. In an embodiment, the amount of the resistant starch in one serving is less than 25 g, less than 20 g, less than 15 g, less than 12.5 g, less than 10 g, less than 9 g, less than 8 g, less than 7 g, less than 6 g or less than 5 g.

In an embodiment of the invention, the inulin-type fructan is present in an amount of at least 0.1 wt.%, based on the total dry weight of the dietetic product, more preferably at least 0.25 wt.%, at least 0.5 wt.%, at least 0.75 wt.%, at least 1 wt.%, at least 1.25 wt.%, at least 1.5 wt.%, at least 1.75 wt.%, at least 2 wt.%, at least 2.25 wt.% or at least 2.5 wt.%. In an embodiment of the invention, the inulin-type fructan is present in an amount of less than 20 wt.%, based on the total dry weight of the dietetic product, more preferably at less than 10 wt.%, less than 9 wt.%, less than 8 wt.%, less than 7.5 wt.%, less than 7 wt.%, less than 6.5 wt.%, less than 6 wt.%, less than 5.5 wt.%, or less than 5 wt.%.

In an embodiment of the invention, the resistant starch is present in an amount of at least 0.1 wt.%, based on the total dry weight of the dietetic product, more preferably at least 0.25 wt.%, at least 0.5 wt.%, at least 0.75 wt.%, at least 1 wt.%, at least 1.25 wt.%, at least 1.5 wt.%, at least 1.75 wt.%, at least 2 wt.%, at least 2.25 wt.% or at least 2.5 wt.%. In an embodiment of the invention, the resistant starch is present in an amount of less than 20 wt.%, based on the total dry weight of the dietetic product, more preferably at less than 10 wt.%, less than 9 wt.%, less than 8 wt.%, less than 7.5 wt.%, less than 7 wt.%, less than 6.5 wt.%, less than 6 wt.%, less than 5.5 wt.%, or less than 5 wt.%.

A particularly preferred dietetic product, in accordance with the invention is a product in the form of an edible bar, such as a nutritional bar, energy bar, diet bar or food supplement bar, snack bar, etc., examples of which are well known to those of skill in the art. Hence, in an embodiment of the invention, a dietetic product as defined herein is provided, wherein the dietetic product is an edible bar, said bar comprising an edible solid or semi-solid bar-shaped, e.g. cuboid or cylindrical, body, optionally in association with one or more other edible ingredients or compositions, e.g. in the form of an edible coating and/or layers covering part of the surface of said edible body. In a preferred embodiment of the invention, said edible body comprises the dietary fiber composition as defined herein. In one embodiment of the invention, the edible body is a cereal composition comprising a cereal mix and a binding syrup. The binding syrup can include e.g. glucose syrup, granulated sugar, glycerol, water, emulsifier, fat and flavors. The dietary fiber composition of the invention can suitably be incorporated in the binding syrup. The binding syrup may be heated along with the inulin and the resistant starch or they may be added along with the cereal components once the syrup is cooled. Non-limiting examples of preferred cereals that can be applied in the cereal mix are chosen from the group consisting of rice crisps, oat flakes, wheat flakes, barley flakes, and combinations thereof. The cereal mix can further contain other components like dried nuts and dried fruit pieces such as dried peaches, apricots, orange rind, apple and raisins. The cereal mix may further comprise insoluble dietary fiber particles. In a preferred embodiment, the cereal composition comprises between 45 and 70 wt% of a cereal mix and between 30 and 55 wt% of binding syrup, wherein the weight percentages are based on the weight of the edible body. In a more preferred embodiment, the cereal composition comprises between 55 and 65 wt% of a cereal mix and between 35 and 45 wt% of binding syrup. Products of this type are known by those skilled in the art, e.g. from international patent publication no. WO 2017/078519.

Except in the examples, or where otherwise expressly indicated, all numerical quantities in this description indicating amounts of material or conditions of reaction and/or use are to be understood as modified by the word "about" in describing the broadest scope of the invention. Practice within the numerical limits stated is generally preferred. Also, unless expressly stated to the contrary: percent, "parts of," and ratio values are by weight; the description of a group or class of materials as suitable or preferred for a given purpose in connection with the invention implies that mixtures of any two or more of the members of the group or class are equally suitable or preferred; description of constituents in chemical terms refers to the constituents at the time of addition to any combination specified in the description, and does not necessarily preclude chemical interactions among the constituents of a mixture once mixed; the first definition of an acronym or other abbreviation applies to all subsequent uses herein of the same abbreviation and applies mutatis mutandis to normal grammatical variations of the initially defined abbreviation; and, unless expressly stated to the contrary, measurement of a property is determined by the same technique as previously or later referenced for the same property.

It is also to be understood that this invention is not limited to the specific embodiments and methods described below, as specific components and/or conditions may, of course, vary. Furthermore, the terminology used herein is used only for the purpose of describing particular embodiments of the present invention and is not intended to be limiting in any way.

It must also be noted that, as used in the specification and the appended claims, the singular form "a," "an," and "the" comprise plural referents unless the context clearly indicates otherwise. For example, reference to a component in the singular is intended to comprise a plurality of components.

Throughout this application, where publications are referenced, the disclosures of these publications in their entireties are hereby incorporated by reference into this application to more fully describe the state of the art to which this invention pertains.

### Description of Figures

Figure 1: Hydrogen (H₂) breath analysis: Fasting (t=0), postprandial first two hours (t=0-120) and last two hours (t=120-240) of H₂-breath for lean (A) and obese (B) subjects. Values are means ± standard deviations. *P<0.05
Figure 2: Energy Expenditure and Substrate Oxidation: Energy expenditure of lean (A) and obese (B), carbohydrate oxidation of lean (C) and obese (D), and lipid oxidization of lean (E) and obese (F) subjects. Values are means ± standard deviations. *P<0.05
Figure 3: Plasma glucose concentrations: Plasma glucose concentrations of lean (A) and obese (B) subjects. Values are means ± standard deviations. *P<0.05

### Examples

### Example 1: Increased acetate concentrations in in vitro model study

The *in vitro* fermentation colon model, TIM-2, was inoculated with pooled fecal microbiota (basically as described in Kortman GA, Dutilh BE, Maathuis AJ, Engelke UF, Boekhorst J, Keegan KP, Nielsen FG, Betley J, Weir JC, Kingsbury Z, Kluijtmans LA, Swinkels DW, Venema K, Tjalsma H. Front Microbiol. 2016, 6:1481) samples from from 11 'metabolically healthy' subjects. The fecal microbiota was freshly sampled and directly (within 2h) placed on ice and under anaerobic conditions. Next, in an anaerobic cabinet, samples were diluted 1:1 with dialysate, and pooled at approximately equal weight, after which glycerol was added and aliquots (30 ml/tube) were snap-frozen in liquid nitrogen and stored in the -80 freezer.

Prior to inoculation, the 4x 30-ml aliquots were taken from the -80 freezer and thawed in a water bath at 37°C for exactly 1 hour. In an anaerobic cabinet, the microbiota from the 4 tubes were combined in 1 beaker, the same volume of pre-reduced dialysate was added, and from there divided over 4 syringes of 60 ml with each ca. 60 ml of microbiota-containing liquid. The syringes were sealed with a small flexible tube with a yellow scissor. Each TIM-2 unit was inoculated with 1 of the 4 syringes (i.e. 60 ml microbiota/dialysate mixture). (There is only a single sample port through which TIM-2 is inoculated). After the microbiota was introduced in the unit, another 60 ml of pre-reduced dialysate was added to get to the final volume of 120 ml of the system.

To simulate the conditions in the proximal region of the colon, the colon transversum and the distal part of colon, the pH was increased throughout the protocol (pH 5.8-7.0). This simulated the traffic of fibers through the colon within a 24 hours experiment (whereas the last 14 hours simulated the more distal colonic site). Mixing was done by peristaltic movements as described in Minekus, M (1998. Development and validation of a dynamic model of the gastrointestinal tract. PhD thesis, Delft University of Technology, The Netherlands).

Samples were taken for SCFA production analysis (performed at Brightlabs B.V., Venlo, The Netherlands), according to) Sayago-Ayerdi SG, Zamora-Gasga VM, Venema K, Food Research International, 13 December 2017:
The dietary fibers (7.5 g of fibers or 7.5 g of fibers + 7.5 g of RS) were added through the sample port, after a 40-hour adaptation period. An experimental week contained the following steps:
- Monday: Start up all 4 units (pH 5.8);
- Tuesday: Feeding of Simulated ileal environment medium (SIEM) (Polzin S, Huber C, Eylert E, Elsenhans I, Eisenreich W and Schmidt H. Appl Environ Microbiol. 2013, 79:3703-3715);
- Wednesday: 3 h starvation period + single shot of test products; Throughout the day sampling after 1, 2, 4, 6 and 8 h;
- Thursday: 24 h after addition of shot: last sample for analysis;
- Friday: cleaning.

The fibers tested were: inulin (Frutafit®TEX!, ex Sensus b.v., The Netherlands), with and without RS.

The increase in cumulative absolute acetate amount between the last two sampling points was taken as an indication for the potential increase in acetate in the distal colon:

| **Fiber added** | **SCFA production (mmol)** |
|---|---|
| Inulin without RS | 8,4 |
| Inulin with RS | 13,7 |

Addition of inulin showed an increase in acetate production in the last 14-16 hours (simulating production in the distal colon).

Addition of inulin showed an additional increase in acetate production in the last 14-16 hours (simulating production in the distal colon) when RS was co-incubated.

### Example 2: A double blind, placebo-controlled, randomized, crossover intervention study with inulin

The study is a double blind, randomized crossover study, which allows evaluation of the role of mixtures of indigestible carbohydrates on fecal and plasma acetate concentrations and substrate and energy metabolism in 24 male subjects. After initial screening, the subjects obtain their intervention supplement 3 times with at least 14-days washout in between. The day before each visit, participants consume one of the supplements with standardized meals.

As study population of twelve lean (BMI ≥ 20kg/m² and ≤ 24.9kg/m²) healthy men aged 30 - 65 years and 12 overweight/obese (BMI ≥ 25kg/m² and ≤ 34.9kg/m²) pre-diabetic men aged between 30 - 65 years are selected. The lean, normoglycaemic individuals: have normal fasting glucose levels (plasma glucose < 6.1 mmol/L) and glucose tolerance (2h glucose after OGTT) (plasma glucose < 7.0 mmol/L). The overweight/obese prediabetic individuals: have impaired fasting glucose levels (plasma glucose ≥ 6.1 mmol/L and ≤ 7.0 mmol/L) and/or impaired glucose tolerance (plasma glucose ≥ 7.8 mmol/L and ≤ 11.0 mmol/L). Both groups consist of Caucasian males, aged 30 - 65 years, with normal blood pressure (systolic blood pressure 100-140mmHg, diastolic blood pressure 60-90 mmHg) and a stable weight for at least 3 months (± 2 kg).

The intervention study is set-up as follows. The day before each visit, participants consume one of the supplements with standardized meals:
- Placebo: 11.43 g (3 x 3.81 g) maltodextrin Glucidex IT 12 (Roquette Freres, Lestrem, France) (43.434 kcal/d)
- Inulin: 12 g (3 x 4 g) inulin Frutafit® TEX! (Sensus B.V., Roosendaal, The Netherlands) in combination with 5.43 g (3 x 1.81 g) maltodextrin to make it isocaloric (43.434 kcal/d).
- Inulin with RS: 12 g (3 x 4 g) of the dietary fibre inulin Frutafit® TEX! (Sensus B.V., Roosendaal, The Netherlands in combination with 9.375 g (3 x 3.125 g) granular potato starch (Avebe, Veendam, The Netherlands) (80% resistant starch (3 x 2.5 g)) (43.434 kcal/d).
All the 3 products are therefore isocaloric.

Subjects consume the supplements with breakfast, lunch and a standardized diner by (1) transferring the inulin/placebo content of the sachets in a cup, mixing with approximately 200 ml hot tea and consuming the complete drink; and (2) transferring the resistant starch/placebo content of the sachets in a 150-ml yoghurt and consume the complete yoghurt. As primary study parameters/endpoints the effects of acute supplementation of inulin alone or in a mixture with RS on plasma and faecal acetate availability in two different metabolic phenotypes (lean, normoglycemic vs. overweight/obese, prediabetic men) are determined.

As secondary parameters, the following are determined:
- Energy expenditure, fat and carbohydrate oxidation measured using an open-circuit ventilated hood system (Omnical, Maastricht University, The Netherlands);
- Circulating hormone concentrations (Insulin, GLP-1, PYY);
- Circulating metabolite concentrations (Glucose, Free Fatty Acids, Triglycerides);
- Inflammatory markers (TNF-α, IL-6, IL-1β, Adipokines);
- Appetite (Visual Analog Scales (VAS)-scoring system for hunger and satiety);
- Breath H₂ using (Bedfont EC60 Gastrolyzer, Rochester, UK);
- Faecal microbiota composition (16S rRNA);
- Three-day food record (a three-day food record was completed three days prior to each Clinical Investigation Day, CID);
- Gastrointestinal Symptom Rating Scale (GSRS) questionnaire (this was completed the day before the CID (=the day when supplementation with the intervention products occurs), the day of the CID, and the day after the CID, and Bristol Stool Scales.

Before the three CIDs (approximately 5 hours) the subjects had a 10-hour fasting period. During baseline conditions, and after the high-fat test meal, plasma acetate concentration and markers of substrate and energy metabolism is determined. Also, energy expenditure, fat and carbohydrate oxidation (open-circuit ventilated-hood system) was assessed, venous blood was taken, H₂ breath and VAS scores were assessed.

The results confirm that the combination of inulin with RS has a beneficial effect on acetate levels in the distal part of the colon, compared to control and that the combination favorably affects secondary parameters. The combination does so more effectively than inulin alone. Altogether the results indicate that the treatments of the present invention will be effective in managing weight and/or in the treatment or prevention of obesity as well as in treatment of other disorders which may benefit from increased acetate availability in the distal part of the colon.

### Example 3: A double blind, placebo-controlled, randomized, crossover intervention study with inulin

Based on the results obtained with the *in vitro* model of the human colon (see example 1), a human intervention study was designed. In this *in vivo* study lean, normoglycaemic vs. obese, prediabetic men are supplemented with inulin alone or a mixture with resistant starch to study effects on fasting and postprandial substrate and energy metabolism.

### Methods

*Participants:* This study was designed as a double blind, placebo-controlled, randomized, crossover study. The study population consisted of 12 normoglycemic lean (BMI ≥ 20kg/m² and ≤ 24.9kg/m²) healthy men aged 30 - 65 years and 11 overweight/obese (BMI ≥ 25kg/m²and ≤ 34.9kg/m²) pre-diabetic men aged between 30 - 65 years. Every volunteer was screened before entering the study for his eligibility. In order to be eligible to participate in this study, an individual must meet either of the following criteria: lean, normoglycemic individuals had to have a BMI between 20 kg/m² and 24.9 kg/m² with normal fasting glucose (plasma glucose < 6.1 mmol/L) and glucose tolerance (2h glucose after oral glucose tolerance test, OGTT) (plasma glucose < 7.8 mmol/). Overweight/obese prediabetic individuals had to have a BMI between 25 kg/m2 and ≤ 34.9 kg/m2 and impaired fasting glucose (plasma glucose ≥ 6.1 mmol/L and ≤ 7.0 mmol/L) and/or impaired glucose tolerance (plasma glucose ≥ 7.8 mmol/L and ≤ 11.0 mmol/L). All participants had to be Caucasian, have a normal blood pressure (systolic blood pressure 100-140mmHg, diastolic blood pressure 60-90 mmHg), and had to be weight stable for at least 3 months (± 2 kg).

### Study design

*Screening:* During the screening day, after an overnight fast, the volunteers underwent the following standard measurements: Body weight, height, waist-to-hip ratio (WHR), blood pressure (diastolic and systolic) Additionally, a fasting blood sampling (20ml) was taken for the determination of plasma glucose and insulin concentration and alanine-aminotransferase (ALAT), creatinine, and haemoglobin A1c (HbA1c) concentration. Two hours after a 75g glucose drink (OGTT) another blood sample (5ml) was taken for analyzing plasma glucose. Furthermore, subjects completed a health questionnaire, including questions about their medical history including history of cardiovascular diseases, gastrointestinal diseases, family history of diabetes, asthma and medicine use.

### Intervention

The intervention of this study was the consumption of one of three supplements, inulin, inulin with resistant starch, or placebo, which were all isocaloric. **1. Inulin (INU):** 12 g (3 x 4 g) inulin Frutafit®TEX! (Sensus B.V., Roosendaal, The Netherlands) in combination with 5.43 g (3 x 1.81 g) maltodextrin to make it isocaloric (43.434 kcal/d). **2. Inulin with resistant starch (INU+RS):** 12 g (3 x 4 g) of the dietary fibre inulin Frutafit® TEX! (Sensus B.V., Roosendaal, The Netherlands in combination with 9.375 g (3 x 3.125 g (80% resistant starch (3 x 2.5 g)) granular potato starch (Avebe, Veendam, The Netherlands) (43.434 kcal/d). **3. Placebo (PLA):** 11.43 g (3 x 3.81 g) maltodextrin Glucidex IT 12 (Roquette Freres, Lestrem, France) (43.434 kcal/d) All the 3 products are therefore isocaloric. The indigestible carbohydrate mixtures and placebo were provided to the study participants in sachets. All intervention products were packed at the food-grade kitchen of the Metabolic Research Unit Maastricht by an independent, experienced researcher. Blinding was ensured by the fact that the content and packaging of the intervention products and placebo sachets looked identical. The sachets were stored at room temperature.

### Clinical investigation day

The main measurements focused on the effects of acute (1 day, 3 times ingestion) supplementation of inulin alone or in a mixture with resistant starch on plasma and faecal acetate availability and postprandial substrate metabolism the day after (CID) in the two different metabolic phenotypes (lean, normoglycemic vs. overweight/obese, prediabetic men).

Participants were instructed to take two prepared sachets of the provided supplements with every meal on the day before their clinical investigation day (CID). They ingested the intervention products with breakfast, lunch and a standardized diner (Aviko Maaltijdpannetje malse kipfilet 450g) by transferring the inulin/placebo content of the sachets in a cup, mixing with approximately 200 ml hot tea and consuming the complete drink and transferring the resistant starch/placebo content of the sachets in a provided cup of yoghurt (Mondelice Paradijs roomdessert 150 ml) and consuming the complete yoghurt. To screen theirfood intake before the CIDs, participants filled in a three-day food questionnaire before each CID. In addition, subjects were instructed to refrain from food products rich in dietary fibers the day before the CIDs.

All participants arrived after an overnight fast (12 hours) on the CID by public transport or car, to prevent as much physical activity as possible, at the Metabolic Research Unit Maastricht. The parameters measured at the CID were energy expenditure, fat and carbohydrate oxidation, measured by using an open circuit ventilated hood system (Omnical, Maastricht University, The Netherlands) and breath H₂ using (Bedfont EC60 Gastrolyzer, Rochester, UK). The latter was used as a marker for colonic bacterial fermentation, which indicates the production of acetate. These measurements were carried out during baseline conditions, and after a liquid high-fat test meal, H₂ breath was assessed and blood was drawn before and 30, 60, 120, 180 and 240 minutes after the high fat mixed meal intake. The liquid test meal provides 2.6 MJ, consisting of 61 E% fat (35.5 E% saturated fat, 18.8 E% monounsaturated fat, and 1.7 E% polyunsaturated fat), 33E% carbohydrates and 6.3 E% protein.

### Indirect calorimetry, formulas, and hydrogen breath analysis

Energy Expenditure, fat and carbohydrate oxidation were measured using an open-circuit ventilated hood system (Omnical, Maastricht University, The Netherlands). VCO2 (L/min) and VO2 (L/min) were measured half an hour before and the four hours after the high-fat mixed meal. The equations of Weir and Frayn ^{32, 33} were used to calculate resting metabolic rate (RMR) and the total rate of fat and carbohydrate oxidation. Nitrogen (N) excretion was calculated based on the assumption that protein oxidation represents 15% of total energy expenditure. Hydrogen (H₂) breath concentration (ppm) was measured every 30 minutes using a Bedfont EC60 Gastrolyzer (Rochester, UK).

### Plasma glucose

Blood was collected into ice-cold EDTA tubes (0.2M EDTA (Sigma, Dorset, UK)). The samples were centrifuged at 3,000 g, 4 °C for 15 minutes, plasma was aliquoted and directly snap-frozen in liquid nitrogen and stored at - 80 °C until analysis. Plasma glucose was measured with enzymatic assays on an automated spectrophotometer (ABX Pentra 400 autoanalyser, Horiba ABX, Montpellier, France).

### Statistical analysis

Values are expressed as mean ± SD. Lean versus obese population baseline values were analyzed using an independent t-test. Responses after indigestible carbohydrate mixtures and placebo supplementation on fasting (t0) and postprandial (t0 - t240) energy expenditure, substrate oxidation, plasma glucose concentrations and H₂ breath were assessed. The postprandial conditions were expressed as total area under the curve (AUC0-240), or divided in periods of 2h (AUC0-120 and AUC 120-240), which were calculated by using the trapezoid method. Differences in fasting (t0) and postprandial AUC between the three interventions (placebo, inulin, and inulin + resistant starch) were analyzed using a linear mixed model for repeated measures. Intervention was set as fixed factor and participants was set as random factor. Although no carry-over effects were expected due to the 14-day washout period, we tested for carry-over effects by adding "period" (order of treatments) to the model as fixed factor and tested for its significance. No carry-over effect was present in the assessed parameters. All statistics were performed using SPSS 25.0 for Windows (UK) and p < 0.05 (two-sided p-value) was considered statistically significant.

### Results

Twelve healthy lean and twelve obese normoglycemic men were included in this intervention study. The lean participants were on average 54 years old, had an average BMI of 23.6 kg/m² and mean fasting glucose was 5.12 mmol/L. The obese study population had an the average age of 59 years, BMI of 29.2 kg/m², and fasting glucose level of 5.97 mmol/L. Baseline characteristics of all volunteers are presented in table 1. No adverse events occurred during this trial. One subject in the obese group decided to not continue the study (dropout) after his first CID due to personal circumstances.

**Table 1. Participants' baseline characteristics**

| Variables | Lean (n=12) | | Obese (n=11) | | p-value |
|---|---|---|---|---|---|
| | Mean | SD | Mean | SD | |
| Age, years | 54 | 12 | 59 | 7 | 0.197 |
| Height, cm | 1.76 | 0.08 | 1.79 | 0.06 | 0.398 |
| Weight, kg | 73.5 | 9.3 | 93.3 | 10.3 | 0.000 |
| Body Mass Index, kg/m² | 23.6 | 1.4 | 29.2 | 2.8 | 0.000 |
| Waist | 86.8 | 7.2 | 107.3 | 9.0 | 0.000 |
| Hip | 96.3 | 6.3 | 107.1 | 7.3 | 0.001 |
| Waist-Hip Ratio | 0.90 | 0.05 | 1.00 | 0.05 | 0.000 |
| Systolic blood pressure, mm Hg | 125 | 11 | 131 | 13 | 0.227 |
| Diastolic blood pressure, mm Hg | 79 | 7 | 79 | 8 | 0.828 |
| Fasting glucose, mmol/L | 5.12 | 0.23 | 5.97 | 0.75 | 0.003 |
| OGTT 2h glucose, mmol/L | 4.78 | 1.33 | 8.10 | 2.52 | 0.002 |
| ALAT, U/L | 22 | 7 | 31 | 12 | 0.030 |
| Creatine, µmol/L | 83 | 13 | 82 | 11 | 0.829 |
| HbA1c % | 5.3 | 0.3 | 5.6 | 0.3 | 0.022 |
| HbA1c mmol/mol Hb | 34 | 3 | 3.8 | 4 | 0.004 |
| Values are presented as mean ± standard deviation (SD); BMI, body mass index;; OGTT, oral glucose tolerance test; ALAT, alanine-aminotransferase; Hb1Ac, haemoglobin 1Ac. | | | | | |

### Hydrogen (H₂) breath analysis

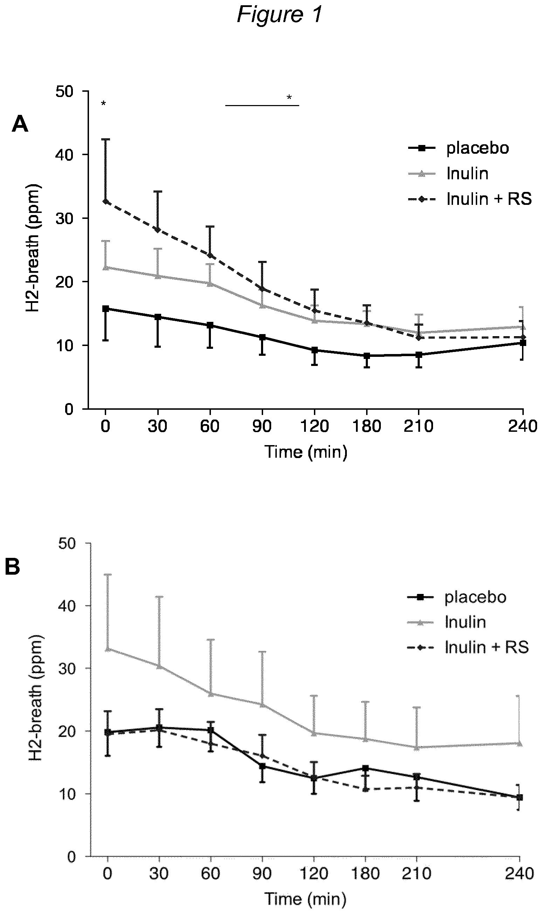

In the lean group that consumed INU+RS, fasting (P=0.019) and postprandial (AUC0240, P=0.009) hydrogen breath analysis was significantly higher as compared to PLA (figure 1A). In the obese population no differences between the interventions in both fasting- and postprandial hydrogen breath analysis were found (figure 1B).

### Substrate Oxidation and Energy Expenditure

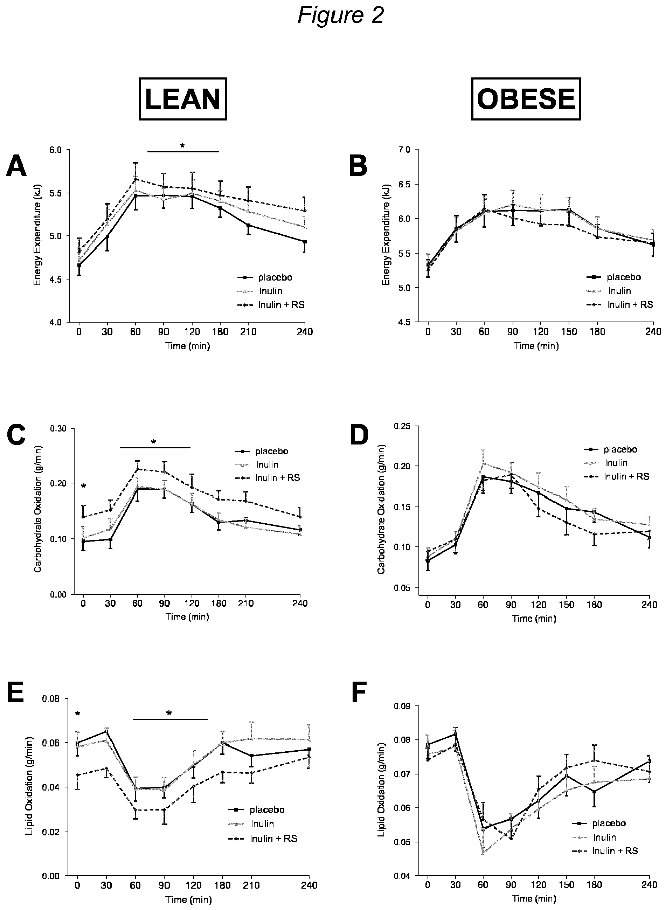

In the lean group there was an increase in postprandial energy expenditure when the subjects were supplemented with INU+RS compared to PLA (P=0.02, figure 2a). Fasting carbohydrate oxidation was increased after INU+RS intake as compared to PLA (P=0.025) and postprandial carbohydrate oxidation was increased in INU+RS compared to PLA and INU (P=0.042 and P=0.045, respectively, figure 2c). Fasting (P=0.034) as well as postprandial (P=0.42) lipid oxidation were decreased when the lean individuals were supplemented with INU+RS compared to PLA (figure 2e).

No significant differences in resting and postprandial energy expenditure and fasting and postprandial substrate oxidation were detected between treatment groups in the obese phenotype (figure 2b,d,f).

### Plasma glucose concentrations

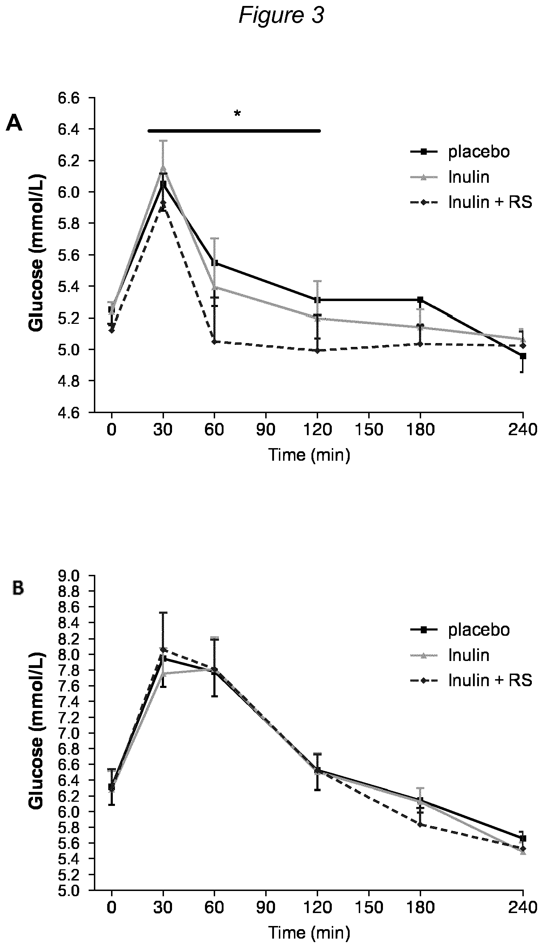

Only INU+RS decreased postprandial glucose levels as compared to PLA (AUC0-120, P<0.05, figure 3a) in the lean phenotype. In contrast, no significant treatment effects on plasma glucose levels were found in the obese phenotype (figure 3b).

### Discussion

In this double blind, placebo-controlled, randomized, crossover study the acute effects of one day supplementation with a slowly fermentable, long-chain inulin, long-chain inulin combined with a rapidly fermentable resistant starch, and placebo on hydrogen breath excretion, postprandial energy expenditure and substrate metabolism the morning after the supplementation day were assessed in lean and pre-diabetic overweight/obese male participants. In the lean group, an increase in hydrogen breath excretion during fasting and postprandial conditions, an increase in energy expenditure and carbohydrate oxidation, and an improved glucose tolerance with inulin combined with resistant starch as compared to placebo were demonstrated. In contrast, there were no significant differences in hydrogen breath, energy expenditure and substrate metabolism between treatment groups in the obese, prediabetic individuals.

The observed effects on hydrogen breath excretion are in accordance with the findings in the *in vitro* TIM-2 model of the human colon. In the TIM-2 model, it was found that combining resistant starch with a long-chain inulin enhanced distal colonic acetate production in particular in the lean phenotype as compared to INU, whereas in obese no increase compared to INU was observed. In line, in the present study it was identified that INU+RS increased hydrogen breath excretion during fasting and postprandial conditions compared to PLA in lean but not in obese individuals. As hydrogen in the breath is an important indicator for gut bacterial fermentation, the initial *in vitro* findings of a more pronounced distal colonic fermentation in the lean as compared to the obese phenotype seem to translate into in vivo conditions.

Interestingly, only in the lean phenotype an increase in energy expenditure and carbohydrate oxidation, and an improved glucose tolerance was observed when resistant starch combined with a long-chain inulin was supplemented the day before, indicating that these effects may be driven by an increased gut microbial fermentation.

These data strongly suggest the involvement of SCFA in these processes, since SCFA are the major metabolic end products of the carbohydrate fermentation. SCFA, and in particular the most abundant SCFA acetate, have been shown to improve host energy expenditure, insulin sensitivity and glucose tolerance in several animal as well as human studies. The underlying mechanisms involve SCFA induced gut-brain signalling pathways thereby including increased secretion of gut hormones and intestinal gluconeogenesis as well as direct effects of SCFA on peripheral metabolism in tissues like the adipose tissue and skeletal muscle.

The reason why intervention effects were only observed in lean individuals but not in the obese, prediabetic phenotype may be explained by differences in initial microbiota composition and SCFA metabolism/handling in peripheral tissues. In the obese individuals, there might be less bacterial species with the capacity to ferment long-chain inulin like for instance specific species of bifidobacteria, which have the capacity to grow on long-chain inulin. This suggests that a more prolonged consumption of the fiber mixture might be essential to increase distal colonic fermentation of long-chain inulin and improve metabolic health in the obese prediabetic individuals.

In conclusion, the data showed that a 1 day supplementation of an unique fiber mixture of resistant starch and long-chain inulin increased colonic bacterial fermentation, the morning after the supplementation day, as compared to either long-chain inulin alone or placebo in lean but not in obese prediabetic individuals. This enhanced bacterial fermentation markedly increased energy expenditure and carbohydrate oxidation during baseline and postprandial conditions. A more prolonged period of supplementation is expected to induce similar effects in obese prediabetic individuals. This indicates that the mixture is a potent tool to prevent the onset of obesity and associated insulin resistance and T2DM.

## Claims

1. Dietary fiber composition comprising a combination of (a) at least one inulin-type fructan; and (b) resistant starch; for use in a method of prophylactic and/or curative treatment of a disease or condition in a subject in need thereof, said disease or condition being selected from the group of obesity, complications associated with obesity, hyperglycemic conditions, metabolic syndrome, overweight, excess body fat (adiposity), diabetes type 2 and prediabetes.

2. Dietary fiber composition for use according to claim wherein the at least one inulin-type fructan (a) is long-chain inulin, preferably a long-chain inulin having an average degree of polymerization of at least 15.

3. Dietary fiber composition for use according to claim 1, wherein the at least one inulin-type fructan (a) is short chain inulin, preferably a short-chain inulin having an average degree of polymerization within the range of below 15.

4. Dietary fiber composition for use according to claim 1, wherein the at least one inulin-type fructan (a) is native inulin.

5. Dietary fiber composition for use according to any one of the preceding claims, wherein the resistant starch (b) is selected from the group consisting of RS2 and RS3.

6. Dietary fiber composition for use according to any one of the preceding claims, wherein the resistant starch (b) is **characterized by** an amylose content, based on the total dry weight of the starch, of at least 35 wt.%.

7. Dietary fiber composition for use according to any one of the preceding claims, wherein the inulin-type fructan (a) and the resistant starch (b) are present in a ratio (w/w) within the range of 1/5 to 10/1, preferably 1/2 to 5/1, more preferably 1/1to 2/1.

8. Dietary fiber composition for use according to any one of the preceding claims, wherein the treatment comprises administration of the dietary fiber composition at least once a week, preferably at least once every 3 days, more preferably at least once every two days, most preferably at least once daily.

9. Dietary fiber composition for use according to any one of the preceding claims, wherein the treatment comprises the administration of the inulin-type fructan in an average daily amount of 4-16 g and the administration of the resistant starch in an average daily amount of 4-12 g.

10. Dietary fiber composition for use according to any one of the preceding claims, wherein the subject is a healthy subject, a non-overweight subject or a subject having a body-mass index of less than 25.

11. Non-medical method of controlling body weight, losing body weight, reducing body weight, preventing body weight gain, limiting body weight gain, inducing body weight loss, increasing body weight loss, managing body weight and/or maintaining a healthy body weight, in a healthy subject, said method comprising the administration of a dietary fiber composition comprising a combination of (a) at least one inulin-type fructan and (b) resistant starch or the simultaneous or sequential administration of (a) at least one inulin-type fructan and (b) a resistant starch.

12. Dietary fiber composition comprising a combination of (a) at least one inulin-type fructan; and (b) resistant starch, wherein the at least one inulin-type fructan (a) is long-chain inulin.

13. Dietary fiber composition according to claim 12, wherein the composition comprises the inulin-type fructan (a) and the resistant starch in a ratio (w/w) within the range of 1/5 to 10/1, preferably 1/2 to 5/1, more preferably 1/1to 2/1.

14. Dietetic product selected from the group consisting of snacks, snack bars, a nutritional bars, energy bars, cereal bars, bars for diabetics, meal replacements, ready meals, savory snacks, bakery products, dessert-type products, pasta, nutritional drinks, sports drinks, and liquid meal replacers, comprising a dietary fiber composition as defined in anyone of claims 12 or 13.

15. Dietetic product according to claim 13, wherein the dietary product is in the form of individually packaged single servings, each serving comprising 2-8 g of the inulin component (a) and/or comprising 1-6 g of the resistant starch (b).
